# EUROPEAN PATENT SPECIFICATION

(11) **EP 3 675 819 B1**
(45) Date of publication and mention of the grant of the patent: **14.01.2026**
(21) Application number: 18797094.2
(22) Date of filing: 28.08.2018
(51) Int. Cl.: A61K 9/00, A61K 9/08, A61K 47/18, A61K 47/26, A61K 31/198, A61K 31/519, A61K 31/51, A61P 31/00

(54) **STABLE LIQUID COMPOSITIONS OF PEMETREXED**
STABILE FLÜSSIGE ZUSAMMENSETZUNGEN VON PEMETREXED
COMPOSITIONS LIQUIDES STABLES DE PEMETREXED

(30) Priority: 29.08.2017 IN 201711030501
(43) Date of publication of application: 08.07.2020
(73) Proprietor: Fresenius Kabi Oncology Limited, New Delhi 110066 (IN)
(72) Inventor: KHATTAR, Dhiraj, Gurgaon Haryana 122001 (IN); KHANNA, Rajesh, Gurgaon Haryana 122001 (IN); YADAV, Abhilasha, Gurgaon Haryana 122001 (IN); BHANDARI, Vikas, Gurgaon Haryana 122001 (IN); MODI, Sameer Ramanlal, Gurgaon Haryana 122001 (IN); HEMLATA, Gurgaon 122001 (IN)
(74) Representative: Fresenius Kabi Deutschland GmbH
(86) International application number: PCT/IB2018/056545
(87) International publication number: WO 2019/043569

(56) References cited:
- WO-A1-2013/179248
- WO-A1-2013/179310
- WO-A1-2016/199053
- US-A1- 2015 297 724
- CAVALLARO V. ET AL: "EFFECTS OF THE COMPLEX NANOSPONGES-NAPHTHALENEACETIC ACID AND [Beta] CYCLODEXTRINS ON IN VITRO RHIZOGENESIS OF GLOBE ARTICHOKE", ACTA HORTICULTURAE, no. 983, 1 April 2013 (2013-04-01), pages 369 - 372, XP93069561, ISSN: 0567-7572, DOI: 10.17660/ActaHortic.2013.983.52
- ANONYMOUS: "Methyl-�-cyclodextrin-CD Formulation", SPECIALCHEM, 1 January 2022 (2022-01-01), XP93069559, Retrieved from the Internet <URL:https://cosmetics.specialchem.com/product/i-cd-formulation-methyl-cyclodextrin> [retrieved on 20230802]
- BAKHSGI A K: "Paper No. 1: ORGANIC CHEMISTRY-I (Nature of Bonding and Stereochemistry) Module 13: Cyclodextrins Part-I", 1 January 2022 (2022-01-01), XP93069557, Retrieved from the Internet <URL:http://epgp.inflibnet.ac.in/epgpdata/uploads/epgp_content/S000005CH/P000656/M014915/ET/1515564053CHE_P1_M13_etext.pdf> [retrieved on 20230802]
- ANONYMOUS: "Chelation", Retrieved from the Internet <URL:https://en.wikipedia.org/w/index.php?title=Chelation&oldid=1194426395> [retrieved on 20240109]
- ED VITZ ET AL.: "Chelating Agents", Retrieved from the Internet <URL:https://chem.libretexts.org/Bookshelves/General_Chemistry/ChemPRIME_(Moore_et_al.)/22%3A_Metals/22.10%3A_Chelating_Agents> [retrieved on 20240109]
- ANONYMOUS: "Chelating agent", Retrieved from the Internet <URL:https://www.collinsdictionary.com/dictionary/english/chelating-agent> [retrieved on 20240109]
- ANONYMOUS: "Chekating Agent (Code C360)", Retrieved from the Internet <URL:https://ncit.nci.nih.gov/ncitbrowser/ConceptReport.jsp?dictionary=NCI Thesaurus&code=C360> [retrieved on 20240109]
- ANONYMOUS: "chelating agent", Retrieved from the Internet <URL:https://www.oxfordreference.com/display/10.1093/oi/authority.20110803095605146> [retrieved on 20240109]
- EMA: "Assessment report EMA/523246/2016 (Pemetrexed Actavis) Procedure No. EMEA/H/C/004109/0000", EMA/819302/2015, 19 November 2015 (2015-11-19), pages 1 - 31, XP055540824, Retrieved from the Internet <URL:https://www.ema.europa.eu/documents/assessment-report/pemetrexed-actavis-epar-public-assessment-report_en.pdf> [retrieved on 20190111]

## Description

### FIELD OF THE INVENTION

The present invention relates to a room temperature stable liquid pharmaceutical composition comprising pemetrexed. The invention further relates to a process for manufacturing the composition as well as use of the composition of the invention.

### BACKGROUND OF THE INVENTION

Pemetrexed belongs to the class of chemotherapy drugs called folate antimetabolites and is used in the treatment of malignant pleural mesothelioma and non-small cell lung cancereither as monotherapy or as combination therapy with cisplatin. Pemetrexed has the chemical name N-{4-[2-(2-Amino-4-oxo-4,7-dihydro-1H-pyrrolo[2,3-d]pyrimidin-5-yl)ethyl]benzoyl}-L-glutamic acid and is represented by the chemical formula as formula (I) below, as first disclosed in U.S. patent no. 5,344,932.

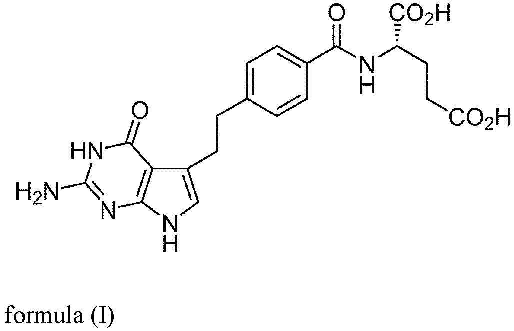

By inhibiting thymidylate synthase (TS), dihydrofolate reductase (DHFR), and glycinamide ribonucleotide formyl transferase (GARFT), and hence the formation of precursor purine and pyrimidine nucleotides, pemetrexed prevents the formation of the DNA and RNA required for the growth and survival of both normal cells and cancer cells.

Pemetrexed disodium salt heptahydrate represented by formula(II) is marketed by Eli Lilly and Company under the trade name ALIMTA^{®} as sterile lyophilized powder for intravenous administration.

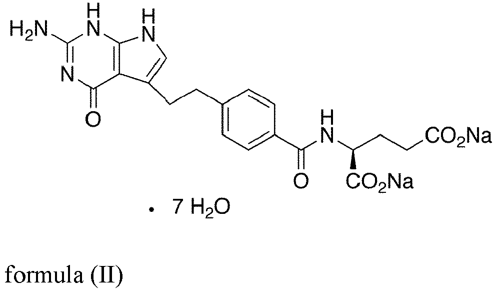

The Eli Lilly commercial product is reported to contain pemetrexed disodium heptahydrate, mannitol, sodium hydroxide, and hydrochloric acid. ALIMTA^{®} is supplied as powder for injection which requires a reconstitution and dilution step before administration to the patient. Chemical and physical stability of reconstituted and infusion solutions of ALIMTA^{®} were demonstrated up to 24 hours following initial reconstitution, when stored at a temperature of 2 to 8°C.

A ready to use infusion solution of pemetrexed diacid or liquid concentrate formulation to be diluted before administration to the patient that could be stored at room temperature for longer period of time is particularly desired for pemetrexed, wherein such ready-to-use compositions provide easier and safer manufacturing and handling during storage and distribution. It is further desirable if the stable pharmaceutical composition can be prepared without the use of freeze drying techniques and eliminating several steps during final administration. The desired liquid formulation can offer enhanced safety for caregiver handling of the cytotoxic materials. Further, a stable, ready-to-use pharmaceutical composition is more acceptable to the patient.

Formulating a liquid composition of pemetrexed or its salts is particularly challenging because of the tendency of pemetrexed and it salts to degrade. This problem is especially exacerbated when pemetrexed or its salts are present in solution, as its degradation or discolouration occurs much more readily due to oxidation or hydrolysis. The degradation on storage of pemetrexed or pemetrexed salts can lead to a significant visible colour change which leads to lesser patient compliance.

Apart from this, another problem observed with the liquid compositions of pemetrexed is precipitation of excipients in the vials during storage. Over time, the increased concentration of degradation products leads to a diminished activity and quality of the product.

Prior art liquid concentrate formulations have suffered from particulate matter in the drug vials which potentially compromises their safety and might necessitate tedious measures like filtration.

This shows, how critical it is to manufacture a stable liquid composition of pemetrexed or its pharmaceutically acceptable salts which is ready to use/dilute. More specifically, it is challenging to produce a room temperature stable liquid product of pemetrexed at large/ commercial scale.

Attempts to prepare stable liquid compositions comprising pemetrexed have been made in the prior art.

US patent no. US 6,686,365 discloses a stable ready-to-use (RTU) formulation of pemetrexed which is developed by using antioxidants/amino acids like L-cysteine, monothioglycerol and thioglycolic acid that can be stored at temperatures above 4°C in a highly concentrated state.

Previous attempts have been made to stabilize pemetrexed with cyclodextrin but could not provide an ideal approach to stabilizing pemetrexed at room temperature for longer storage time periods.

International patent application WO 2013/179310 A1 claims storage stable concentrated aqueous parenteral composition comprising pemetrexed disodium and at least one stability enhancing adjuvant such as cyclodextrin derivatives and method of preparing these compositions. The formulations claimed in WO 2013/179310 A1 are shown to be stable at refrigerate temperature (2 to 8°C). The pemetrexed formulations disclosed in this application were found unstable at room temperature when reproduced.

Chinese patent application CN 102846563 discloses a pemetrexed disodium lyophilized powder, comprising pemetrexed disodium, mannitol, [beta] - cyclodextrin which needs to be reconstituted before administration to the patient.

WO 2016/199053 Al discloses a stable ready to use liquid parenteral pharmaceutical formulation comprising of Pemetrexed diacid, amino acids and water.

US 2015/0297724 A1 discloses a stabilized pemetrexed formulation comprising acetylcysteine as antioxidant and a citrate salt as buffer.

The EMA assessment report of Pemetrexed Actavis discloses a product containing Pemetrexed diacid as an active agent and other ingredients are trometamol, anhydrous citric acid, cysteine hydrochloride monohydrate and water for injection, which needs to be stored at 2-8°C.

WO 2013/179248 A1 discloses pharmaceutical compositions comprising pemetrexed and an organic amine.

None of these prior art approaches provides a satisfactory solution of providing a simple liquid formulation of pemetrexed or its salts which is room temperature stable and prevents the formation of particulate matter in the finished vials.

There is a need to provide further stable formulations of pemetrexed or pemetrexed salts which are room temperature stable and devoid of any particulate matter during storage. It is therefore an object of the present invention to provide formulations of pemetrexed or its salts, that are stable at room temperature and which can be easily manufactured and readily used.

### SUMMARY OF THE INVENTION

The present invention provides stable liquid composition of pemetrexed, wherein the composition is room temperature stable. Thus, in one aspect of the present invention, there is provided a liquid pharmaceutical composition for parenteral administration comprising:
a) pemetrexed diacid, wherein the concentration of pemetrexed diacid is from about 2.5 mg/ml to about 50 mg/ml;
b) tromethamine, wherein the concentration of tromethamine is from about 1 to 150 mg/ml; and
c) cyclodextrin, wherein the cyclodextrin is selected from β-cyclodextrin, hydroxypropyl-β-cyclodextrin, sulfobutylether-β-cyclodextrin, α-cyclodextrin and γ-cyclodextrin and the concentration of cyclodextrin is from about 40 to 500 mg/ml;

In a particular aspect, the composition of the present inventions comprises pemetrexed diacid, tromethamine, cyclodextrin in suitable quantities to stabilize the composition in aqueous solutions. The composition further comprises inert gas selected from argon, helium and nitrogen. Preferably, the inert gas is nitrogen. The compositions of the present invention further comprise optionally other excipients.

The present invention relates to the composition, wherein said the concentration of pemetrexed diacid is from about 2.5 mg/ml to about 50 mg/ml.

In yet another aspect, the invention relates to the composition, wherein the molar ratio of pemetrexed diacid to cyclodextrin is in range of 1:0.1 to 1:10, preferably 1:0.5 to 1:5, such as 1:2 to 1:5.

In another aspect, the present invention relates to the composition comprising;
a) pemetrexed diacid preferably in an amount of 1-50mg,
b) tromethamine preferably in an amount of 1-150 mg, and
c) hydroxypropyl-β-cyclodextrin preferably in an amount of 40-500mg.

The present invention relates to the composition further comprising one or more pharmaceutically acceptable excipients selected from buffer, organic solvent, chelating agent, antioxidant and solubilizer.

In a further aspect, the compositions of the present invention are ready to use or concentrated liquid which can be further diluted with an infusion solution, for example a saline or dextrose solution.

In another aspect, the present invention relates to a process for manufacturing the liquid pharmaceutical composition for parenteral administration comprising the steps of:
a) purging inert gas in the water for injection until the dissolved oxygen content of water is less than 7 mg/L, preferably less than 3 mg/L at 25 °C,
b) dissolving cyclodextrin in water for injection of step a)
c) adding tromethamine to the above solution of step b),
d) adding pemetrexed diacid to the above mixture and dissolving and optionally adjusting the pH of the solution to about 6.0 - 8.0.

In yet another aspect, the present invention relates to the composition that is substantially free from any particulate matter in the sealed container.

The present invention further relates to the use of the composition of the invention for the treatment of malignant pleural mesothelioma and non-small cell lung cancer.

The present invention relates to the composition which is room temperature stable.

Particularly preferred embodiments are set forth in the claims.

### DETAILED DESCIPTION

While this specification concludes with claims particularly pointing out and distinctly claiming that, which is regarded as the invention, it is anticipated that the invention can be more readily understood through reading the following detailed description of the invention and study of the included examples.

The inventors of the present invention found that several stabilization techniques failed to provide sufficient stable liquid compositions of pemetrexed. As the compound has to be administered by infusion techniques, it must be in a readily available form such as ready to dilute in an infusion liquid, or be provided as ready to use infusion solution. The composition must be a clear solution and the active pharmaceutical ingredient must be homogeneously dissolved with other excipients without any precipitation on storage. Another challenge is preparing such aqueous based compositions of pemetrexed which is room temperature stable. In a preferred embodiment the compositions of present invention are substantially free from any particulate matter in the sealed container.

The inventors of the present application have unexpectedly found that pemetrexed formulations containing tromethamine in combination with cyclodextrin have shown room temperature stability to the active pharmaceutical compound even in aqueous formulations.

In particular, the combination of tromethamine with cyclodextrin might have formed a ternary complex with pemetrexed diacid and this complexation reaction would have provided the extra stability to the formulation due to shielding of reactive sites of pemetrexed diacid. Stability is further enhanced by purging nitrogen to the water for injection to be used for preparation of the infusion solution. The inventors of present invention have thus prepared a stable pharmaceutical composition of pemetrexed diacid which is room temperature stable.

One of the advantages of the compositions of the invention is that they have sufficient long-term stability at room temperature which is defined as less than 2 percent of total impurities after at least about 12 months of storage at a temperature of 25 ± 5°C, which indicates sufficient stability upon storage.

As used herein, the term "pemetrexed" includes pemetrexed diacid or pharmaceutically acceptable salts thereof.

As used herein, the term "pemetrexed diacid" refers to a compound named N-{ 4-[2-(2-Amino-4-oxo-4,7-dihydro-1H-pyrrolo[2,3-d]pyrimidin-5-yl)ethyl]benzoyl}-L-glutamic acid, represented by the formula (I). Specifically, the term refers to a multitargeted antifolate that exhibits anticancer effects against various cancers, including malignant pleural mesothelioma and non-small cell lung cancer.

As used herein, the term "pharmaceutically acceptable salt" refers to a salt prepared according to a conventional method known in the art includes but not limited to disodium, dipotassium or ditromethamine salt.

The present invention is directed to room temperature stable liquid pharmaceutical composition for parenteral administration comprising about 2.5 mg/ml to about 50 mg/ml pemetrexed diacid, about 1 to 150 mg/ml tromethamine and about 40 to 500 mg/ml cyclodextrin, wherein the cyclodextrin is selected from β-cyclodextrin, hydroxypropyl-β-cyclodextrin, sulfobutylether-β-cyclodextrin, α-cyclodextrin and γ-cyclodextrin. The composition may further comprise an inert gas. The compositions of the present invention may be ready-to-use or liquid concentrates which are ready-to-dilute with the infusion solutions such as a saline or dextrose solution. The compositions of the present invention are therefore advantageous because they provide enhanced convenience in handling and administration.

The compositions may be presented in a single vial presentation having pemetrexed diacid concentrations in the range of 2.5 to 50 mg/ml, preferably 5-40 mg/ml, more preferably 10-30 mg/ml, of which the preferred concentration is 25 mg/ml.

These pharmaceutical compositions may then be administered via intravenous infusion for use in the treatment of patients suffering from malignant pleural mesothelioma and for second-line treatment of non small cell lung cancer which is the approved indication of pemetrexed.

The stable ready-to-use pharmaceutical composition of pemetrexed diacid is usually solvated in aqueous solvent comprising water for injection. In one embodiment of the present invention, the ready-to-use pharmaceutical composition of pemetrexed diacid has a pH between about 4 and about 9, preferably between about 5 and about 8 and more preferably in the range of about 6.0 and about 8.0. The pH of such ready-to-use pharmaceutical compositions of pemetrexed diacid may be adjusted with a pharmacologically acceptable pH adjusting agent such as an acid, base, buffer or combination thereof. In an embodiment of the present invention the pH adjusting agent is hydrochloric acid or sodium hydroxide, or a combination thereof. The hydrochloric acid or sodium hydroxide may be in any suitable form, such as a 1 N solution.

In certain preferred embodiments of the present invention, the compositions are ready to use or concentrated liquid ready to be diluted with a suitable infusion solution before administration to the patient, wherein the infusion solution is selected from saline or dextrose solution.

The composition comprises tromethamine. The total amount of tromethamine in the present invention is in the range corresponding to 1 to 150 mg/ml, preferably 10 to 40 mg/ml, more preferably 15 to 35 mg/ml.

The composition of the present invention comprises cyclodextrin which is selected from β-cyclodextrin, hydroxypropyl-β-cyclodextrin, sulfobutylether-β-cyclodextrin, α-cyclodextrin and γ-cyclodextrin, preferably hydroxypropyl-β-cyclodextrin. The cyclodextrin derivative is present in an amount from about 40 mg/ml to about 500 mg/ml, preferably from about 100 mg/ml to about 350 mg/ml. More preferably, the concentration of cyclodextrin derivative in the aqueous parenteral compositions according to the invention is from about 200 mg ml to about 300 mg/ml. The preferred molar ratio of pemetrexed diacid to cyclodextrin according to present invention is in range of 1:0.1 to 1:10, more preferably 1:0.5 to 1:5, such as 1:2 to 1:5.

In another embodiment of the present invention, so as to minimize oxidation of the sensitive material it is also desirable to remove headspace oxygen or moisture or both during the formulation or from the sealable vessel as quickly as possible. This may be aided by, for example, purging the water for injection or sealable container with a gas which is substantially oxygen-free, or substantially moisture free, or substantially oxygen and moisture free before, during or after step, or any combination thereof. Inert gas is purged during manufacturing in the water for injection until the dissolved oxygen content of water is less than 7 mg/L, preferably less than 3 mg/L at 25°C. The headspace of product vials is also blanketed with nitrogen to achieve headspace oxygen content less than 8%, preferably less than 2% to minimize degradation during storage.

The gas used for purging the sealable container may be any appropriate inert gas known to those in the art, such as argon, helium or nitrogen, or a mixture thereof. The most preferred inert gas is nitrogen.

Further one or more pharmaceutically acceptable excipients may as well be included in the compositions of the invention, such as but not limited to buffers, organic solvents, chelating agents, antioxidants, preservatives and solubilizers. Buffer including but not limited to citrate, phosphate, arginate, acetate, glutamate, lactobionate, tartarate, and a mixture thereof. Organic solvent including but not limited to glycerol, poly ethylene glycol (PEG 300, PEG 400), propylene glycol (PG), ethanol, dimethyl acetamide (DMA) and a mixture thereof. Chelating agents including but not limited to ethylenediaminetetraacetic acid (EDTA), sodium citrate. Antioxidant including but not limited to L-cysteine, methionine, monothioglycerol, sodium metasulphite, sodium bi sulphite or a mixture thereof. Solubilizers including but not limited to surfactants/emulsifiers such as polysorbates, polyoxyethylated castor oil, lecithine, polymers such as povidone (PVP), poloxamer, and hydrotropes such as sodium benzoate, sodium salicylate, sodium benzene sulphonate, p-amino benzoic acid hydrochloride, procaine hydrochloride, caffeine, sodium alkanoate, sodium p-toluenesulfonate and sodium xylene sulfonate. Antibacterial preservatives including one or more of phenylmercuric nitrate, thiomersal, benzalkonium chloride, benzethonium chloride, phenol, cresol and chlorobutanol.

### The present invention includes inter alia the following aspects:

In a first aspect, the present invention relates to a liquid pharmaceutical composition for parenteral administration comprising:
a) pemetrexed diacid, wherein the concentration of pemetrexed diacid is from about 2.5 mg/ml to about 50 mg/ml;
b) tromethamine, wherein the concentration of tromethamine is from about 1 to 150 mg/ml; and
c) cyclodextrin, wherein the cyclodextrin is selected from β-cyclodextrin, hydroxypropyl-β-cyclodextrin, sulfobutylether-β-cyclodextrin, α-cyclodextrin and γ-cyclodextrin and the concentration of cyclodextrin is from about 40 to 500 mg/ml.

In a second aspect, the present invention relates to the composition according to aspect 1, wherein the composition further comprises an inert gas.

In a third aspect, the present invention relates to the composition according to aspect 2, wherein the inert gas is selected from nitrogen, argon and helium, preferably nitrogen.

In a fourth aspect, the present invention relates to the composition according to aspect 1, wherein the molar ratio of pemetrexed diacid to cyclodextrin is in range of 1:0.1 to 1:10, preferably 1:0.5 to 1:5, such as 1:2 to 1:5.

In a fifth aspect, the present invention relates to the composition according to any of the preceding aspects, further comprising one or more pharmaceutically acceptable excipients selected from buffer, organic solvent, chelating agent, antioxidant and solubilizer.

In a sixth aspect, the present invention relates to the composition according to aspect 5, wherein the buffer is selected from the group consisting of citrate, phosphate, acetate, glutamate, lactobionate, and a mixture thereof.

In one embodiment, the present invention relates to the composition wherein the buffer is citrate buffer.

In another embodiment, the present invention relates to the composition wherein the buffer is phosphate buffer.

In yet another embodiment, the present invention relates to the composition wherein the buffer is acetate buffer.

In another embodiment, the present invention relates to the composition wherein the buffer is glutamate buffer.

In yet another embodiment, the present invention relates to the composition wherein the buffer is glutamate buffer.

In an seventh aspect, the present invention relates to the composition according to aspect 5, wherein the organic solvent is selected from the group consisting of glycerol, poly ethylene glycol (PEG 300, PEG 400), propylene glycol (PG), ethanol, dimethyl acetamide (DMA) and a mixture thereof.

In one embodiment, the present invention relates to the composition wherein the organic solvent is glycerol.

In another embodiment, the present invention relates to the composition wherein the organic solvent is poly ethylene glycol (PEG 300, PEG 400).

In yet another embodiment, the present invention relates to the composition wherein the organic solvent is propylene glycol (PG).

In another embodiment, the present invention relates to the composition wherein the organic solvent is ethanol.

In yet another embodiment, the present invention relates to the composition wherein the organic solvent is dimethyl acetamide (DMA).

In an eight aspect, the present invention relates to the composition according to aspect 5, wherein the chelating agent is selected from the group consisting of ethylenediaminetetraacetic acid (EDTA), sodium citrate and a mixture thereof.

In one embodiment, the present invention relates to the composition wherein the chelating agent is ethylenediaminetetraacetic acid (EDTA).

In another embodiment, the present invention relates to the composition wherein the chelating agent is sodium citrate.

In a ninth aspect, the present invention relates to the composition according to aspect 5, wherein the antioxidant is selected from the group consisting of methionine, sodium metasulphite, sodium bisulphite and a mixture thereof.

In one embodiment, the present invention relates to the composition wherein the antioxidant is methionine.

In another embodiment, the present invention relates to the composition wherein the antioxidant is sodium metasulphite.

In another embodiment, the present invention relates to the composition wherein the antioxidant is sodium bisulphite.

In a tenth aspect, the present invention relates to the composition according to aspect 5, wherein the solubilizer is selected from the group consisting of povidone (PVP), lecithine, sodium benzoate, poloxamer and a mixture thereof.

In a eleventh aspect, the present invention relates to the composition according to any of the preceding aspects for use in treating a subject suffering from malignant pleural mesothelioma and refractory non small cell lung cancer.

In a twelfth aspect, the present invention relates to the composition according to any of the preceding aspects, wherein the composition is ready to use or ready to be diluted with a suitable infusion solution before administration to the patient.

In a thirteenth aspect, the present invention relates to the composition according to aspect 12, wherein the infusion solution is selected from saline or dextrose solution.

In a fourteenth aspect, the present invention relates to the composition according to aspect 1, is substantially free from any particulate matter in the sealed container.

In a fifteenth aspect, the present invention relates to the composition according to any of the preceding aspects comprising;
a) pemetrexed diacid preferably in an amount of 2.5-50mg,
b) tromethamine preferably in an amount of 1-150 mg,
c) hydroxypropyl-β-cyclodextrin preferably in an amount of 40-500mg.

In a sixteenth aspect, the present invention relates to a process for manufacturing the liquid pharmaceutical composition for parenteral administration according to any of the preceding aspects comprising the steps of:
a) purging inert gas in the water for injection until the dissolved oxygen content of water is less than 7 mg/L, preferably less than 3 mg/L at 25 °C,
b) dissolving cyclodextrin in water for injection of step a)
c) adding tromethamine to the above solution of step b),
d) adding pemetrexed diacid to the above mixture and dissolving and optionally adjusting the pH of the solution to about 6.0 - 8.0,

In a seventeenth aspect, the present invention relates to a process according to aspect 20, wherein the inert gas is nitrogen.

In an eighteenth aspect, the present invention relates to a process according to aspect 20, wherein the cyclodextrin is hydroxypropyl-β-cyclodextrin.

In a nineteenth aspect, the present invention relates to a process for manufacturing the liquid pharmaceutical composition for parenteral administration according to aspect 20 comprising the additional following subsequent to steps a)-d):
e) filtering the solution and filling in vials,
f) headspace blanketing with nitrogen and
g) stoppering and sealing the vials.

In a twentieth aspect, the present invention relates to the composition further comprising a preservative selected from the group consisting of phenylmercuric nitrate, thiomersal, benzalkonium chloride, benzethonium chloride, phenol, cresol and chlorobutanol or a mixture thereof.

In another aspect, the formulation can be sterilized by filteration, aseptic processing, termination sterilization, by combination of such techniques, or any other suitable sterilization process known in the art.

### Examples:

The invention is further illustrated by way of the following examples, which in no way should be construed as limiting the scope of the invention. The process of manufacturing of the compositions below was carried at room temperature (25°C)

### Examples 1, 2, 3 and 4:

**Table 1. Composition of pemetrexed injection**

| **Ingredient** | **Quantity per mL** | | | |
|---|---|---|---|---|
| | **Example 1** | **Example 2** | **Example 3** | **Example 4** |
| Pemetrexed diacid | 25 mg | 25 mg | 25 mg | 25 mg |
| Tromethamine | 15 mg | 15 mg | 15 mg | 15 mg |
| HPβCD | 84 mg | 222 mg | 420 mg | 241 mg |
| Tromethamine | q.s. | q.s. | q.s. | q.s. |
| Hydrochloric acid | q.s. | q.s. | q.s. | q.s. |
| Water | q.s. to 1 mL | q.s. to 1 mL | q.s. to 1 mL | q.s. to 1 mL |
| Nitrogen | q.s. | q.s. | q.s. | q.s. |

### - Manufacturing process (For example 1, 2, 3 and 4)

A suitable quantity of water in a manufacturing vessel was taken. Nitrogen was purged into water until the dissolved oxygen content of the water became less than 7 mg/L, preferably less than 3 mg/L. After nitrogen bubbling, the required quantity of HPβCD was added and dissolved. Once a clear solution was obtained, tromethamine was added and dissolved. After addition of tromethamine, pemetrexed diacid was added and allowed to stir until a clear solution was obtained. If required, the pH of solution was adjusted to 6.0 - 8.0 with the help of 10%w/v tromethamine solution or 1%v/v hydrochloric acid solution. The volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 5.

**Table 2. Composition of pemetrexed injection**

| **Ingredient** | **Quantity per mL** |
|---|---|
| Pemetrexed diacid | 25 mg |
| Tromethamine | 15 mg |
| HPβCD | 222 mg |
| Methionine | 1 mg |
| Tromethamine | q.s. |
| Hydrochloric acid | q.s. |
| Water | q.s. to 1 mL |
| Nitrogen | q.s. |

### - Manufacturing process

A suitable quantity of water in a manufacturing vessel was taken. Nitrogen was purged into the water until the dissolved oxygen content of the water became less than 7 mg/L, preferably less than 3 mg/L. After nitrogen bubbling, the required quantity of HPβCD followed by methionine were added and dissolved. Once a clear solution was obtained, tromethamine was added and dissolved. After addition of tromethamine, pemetrexed diacid was added and allowed to stir until a clear solution was obtained. If required, the pH of solution was adjusted to 6.0-8.0 with the help of 10%w/v tromethamine solution or 1%v/v hydrochloric acid solution. The volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 6.

**Table 3. Composition of pemetrexed injection**

| **Ingredient** | **Quantity per mL** |
|---|---|
| Pemetrexed diacid | 25 mg |
| Tromethamine | 15 mg |
| HPβCD | 222 mg |
| Glycerol | 90 mg |
| Tromethamine | q.s. |
| Hydrochloric acid | q.s. |
| Water | q.s. to 1 mL |
| Nitrogen | q.s. |

### - Manufacturing process

A suitable quantity of water in a manufacturing vessel was taken. Nitrogen was purged into water until dissolved oxygen content of water became less than 7 mg/L, preferably less than 3 mg/L. After nitrogen bubbling, required quantity of HPβCD was added and dissolved. Once a clear solution was obtained, tromethamine was added and dissolved. After addition of tromethamine, pemetrexed diacid was added and allowed to stir until clear solution was obtained. Required quantity of glycerol was added and stirred vigorously for proper mixing of solvent system. If required, the pH of solution was adjusted to 6.0-8.0 with the help of 10%w/v tromethamine solution or 1%v/v hydrochloric acid solution. Volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 7:

**Table 4. Composition of pemetrexed injection**

| **Ingredient** | **Quantity per mL** |
|---|---|
| Pemetrexed diacid | 25 mg |
| Tromethamine | 35 mg |
| Citric acid | 10 mg |
| HPβCD | 222 mg |
| Tromethamine | q.s. |
| Hydrochloric acid | q.s. |
| Water | q.s. to 1 mL |
| Nitrogen | q.s. |

### - Manufacturing process

A suitable quantity of water in a manufacturing vessel was taken. Nitrogen was purged into the water until the dissolved oxygen content of the water became less than 7 mg/L, preferably less than 3 mg/L. After nitrogen bubbling, the required quantity of HPβCD and citric acid was added and dissolved. Once a clear solution was obtained, tromethamine was added and dissolved. After dissolution of tromethamine, pemetrexed diacid was added and allowed to stir until a clear solution was obtained. If required, the pH of solution was adjusted to 6.0-8.0 with the help of 10%w/v tromethamine solution or 1%v/v hydrochloric acid solution. The volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 8:

**Table 5. Composition of pemetrexed injection**

| **Ingredient** | **Quantity per mL** |
|---|---|
| Pemetrexed diacid | 25 mg |
| Tromethamine | 15 mg |
| HPβCD | 222 mg |
| Arginine | 10 mg |
| Tromethamine | q.s. |
| Hydrochloric acid | q.s. |
| Water | q.s. to 1 mL |
| Nitrogen | q.s. |

### - Manufacturing process

A suitable quantity of water in a manufacturing vessel was taken. Nitrogen was purged into water until the dissolved oxygen content of the water became less than 7 mg/L, preferably less than 3 mg/L. After nitrogen bubbling, required quantity of HPβCD was added and dissolved. Once a clear solution was obtained, tromethamine and arginine was added and dissolved. Pemetrexed diacid was added and allowed to stir until a clear solution was obtained. If required, the pH of solution was adjusted to 6.0-8.0 with the help of 10%w/v tromethamine solution or 1%v/v hydrochloric acid solution. The volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 9:

**Table 6. Composition of pemetrexed injection**

| **Ingredient** | **Quantity per mL** |
|---|---|
| Pemetrexed diacid | 25 mg |
| Tromethamine | 15 mg |
| HPβCD | 222 mg |
| Potassium dihydrogen phosphate | 10 mg |
| Tromethamine | q.s. |
| Hydrochloric acid | q.s. |
| Water | q.s. to 1 mL |
| Nitrogen | q.s. |

### - Manufacturing process

Suitable quantity of water in a manufacturing vessel was taken. Nitrogen was purged into water until dissolved oxygen content of water became less than 7 mg/L, preferably less than 3 mg/L. After nitrogen bubbling, required quantity of HPβCD was added and dissolved. Once a clear solution was obtained, tromethamine and potassium dihydrogen phosphate was added and dissolved. pemetrexed diacid was added and allowed to stir until clear solution was obtained. If required, the pH of solution was adjusted to 6.0-8.0 with the help of 10%w/v tromethamine solution or 1%v/v hydrochloric acid solution. Volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 10:

**Table 7. Composition of pemetrexed injection**

| **Ingredient** | **Quantity per mL** |
|---|---|
| Pemetrexed diacid | 25 mg |
| Tromethamine | 15 mg |
| HPβCD | 222 mg |
| Methionine | 1 mg |
| Glycerol | 90 mg |
| Tromethamine | q.s. |
| Hydrochloric acid | q.s. |
| Water | q.s. to 1 mL |
| Nitrogen | q.s. |

### - Manufacturing process

Suitable quantity of water in a manufacturing vessel was taken. Nitrogen was purged into water until dissolved oxygen content of water became less than 7 mg/L, preferably less than 3 mg/L. After nitrogen bubbling, required quantity of HPβCD was added and dissolved. Once a clear solution was obtained, tromethamine and methionine was added and dissolved. pemetrexed diacid was added and allowed to stir until clear solution was obtained followed by addition and mixing of glycerol. If required, the pH of solution was adjusted to 6.0-8.0 with the help of 10%w/v tromethamine solution or 1%v/v hydrochloric acid solution. Volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 11:

**Table 8. Composition of pemetrexed injection**

| **Ingredient** | **Quantity per mL** |
|---|---|
| Pemetrexed diacid | 25 mg |
| Tromethamine | 15 mg |
| HPβCD | 222 mg |
| Methionine | 1 mg |
| Glycerol | 90 mg |
| Tromethamine | q.s. |
| Hydrochloric acid | q.s. |
| Water | q.s. to 1 mL |
| Nitrogen | q.s. |
| Terminal sterilization at 121 °C for 15 min | |

### - Manufacturing process

Suitable quantity of water in a manufacturing vessel was taken. Nitrogen was purged into water until dissolved oxygen content of water became less than 7 mg/L, preferably less than 3 mg/L. After nitrogen bubbling, required quantity of HPβCD was added and dissolved. Once a clear solution was obtained, tromethamine and methionine was added and dissolved. Pemetrexed diacid was added and allowed to stir until clear solution was obtained followed by addition and mixing of glycerol. If required, the pH of solution was adjusted to 6.0-8.0 with the help of 10%w/v tromethamine solution or 1%v/v hydrochloric acid solution. Volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed. The sealed vials were terminally sterilized at 121 ⁰C for 15 min.

### Example 12.

**Table 9. Composition of pemetrexed injection**

| **Ingredient** | **Quantity per mL** |
|---|---|
| Pemetrexed diacid | 25 mg |
| Tromethamine | 15 mg |
| Methionine | 1 mg |
| Tromethamine | q.s. |
| Hydrochloric acid | q.s. |
| Water | q.s. to 1 mL |
| Nitrogen | q.s. |

### - Manufacturing process

Suitable quantity of water in a manufacturing vessel was taken. Nitrogen was purged into water until dissolved oxygen content of water became less than 7 mg/L, preferably less than 3 mg/L. After nitrogen bubbling, required quantity of tromethamine and methionine was added and dissolved. Pemetrexed diacid was added and allowed to stir until clear solution was obtained. If required, the pH of solution was adjusted to 6.0-8.0 with the help of 10%w/v tromethamine solution or 1%v/v hydrochloric acid solution. Volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed.

### Example 13.

**Table 10. Composition of pemetrexed injection**

| **Ingredient** | **Quantity per mL** |
|---|---|
| Pemetrexed diacid | 25 mg |
| Tromethamine | 15 mg |
| Glycerol | 90 mg |
| Tromethamine | q.s. |
| Hydrochloric acid | q.s. |
| Water | q.s. to 1 mL |
| Nitrogen | q.s. |

### - Manufacturing process

Suitable quantity of water in a manufacturing vessel was taken. Nitrogen was purged into water until dissolved oxygen content of water became less than 7 mg/L, preferably less than 3 mg/L. After nitrogen bubbling, required quantity of tromethamine was added and dissolved. pemetrexed diacid was added and allowed to stir until clear solution was obtained followed by addition and mixing of glycerol. If required, the pH of solution was adjusted to 6.0-8.0 with the help of 10%w/v tromethamine solution or 1%v/v hydrochloric acid solution. Volume was made up to 100% with water. The drug solution was filtered through a suitable 0.2µ PVDF filter. The filtered solution was filled into vials. The vial headspace was blanketed with nitrogen to achieve headspace oxygen content less than 8%, preferably less than 2%. The vials were stoppered and finally sealed. The sealed vials were terminally sterilized at 121 ⁰C for 15 min.

### Characterization Data :

Stability profiles of the pharmaceutical compositions as mentioned in above Examples 1 to 13 have been summarized below in Table 11 to 23, respectively.

**Table 11. Stability profile of the pharmaceutical composition as mentioned in Example 1**

| **Storage condition** | **Time point** | **Description** | **pH** | **Total impurity (%)** |
|---|---|---|---|---|
| Initial | | Clear colorless solution, free from visible particles | 6.9 | 0.34 |
| 40°C/75% RH | 1 month | Clear colorless solution, free from visible particles | 6.9 | 0.44 |
| | 3 month | Clear light green colored solution, free from visible particles | 7.0 | 0.72 |
| 25°C/60% RH | 1 month | Clear colorless solution, free from visible particles | 6.9 | 0.34 |
| | 3 month | Clear colorless solution, free from visible particles | 7.0 | 0.41 |

**Table 12. Stability profile of the pharmaceutical composition as mentioned in Example 2**

| **Storage condition** | **Time point** | **Description** | **pH** | **Total impurity (%)** |
|---|---|---|---|---|
| Initial | | Clear colorless solution, free from visible particles | 6.9 | 0.31 |
| 40°C/75% RH | 1 month | Clear colorless solution, free from visible particles | 6.8 | 0.53 |
| | 3 month | Clear light green colored solution, free from visible particles | 6.9 | 0.69 |
| | 6 month | Light green color solution, free from visible particles | 6.9 | 1.20 |
| | 1 month | Clear colorless solution, free from visible particles | 6.9 | 0.40 |
| 25°C/60% RH | 3 month | Clear colorless solution, free from visible particles | 6.9 | 0.43 |
| | 6 month | Clear colorless solution, free from visible particles | 6.9 | 0.52 |
| | 12 month | Clear light green color solution free from visible particles | 6.9 | 0.73 |

**Table 13. Stability profile of the pharmaceutical composition as mentioned in Example 3**

| **Storage condition** | **Time point** | **Description** | **pH** | **Total impurity (%)** |
|---|---|---|---|---|
| Initial | | Clear colorless solution, free from visible particles | 7.4 | 0.36 |
| 40°C/75% RH | 1 month | Clear colorless solution, free from visible particles | 7.4 | 0.48 |
| | 3 month | Clear colorless solution, free from visible particles | 7.7 | 0.78 |
| 25°C/60% RH | 1 month | Clear colorless solution, free from visible particles | 7.3 | 0.34 |
| | 3 month | Clear colorless solution, free from visible particle | 7.6 | 0.42 |

**Table 14. Stability profile of the pharmaceutical composition as mentioned in Example 4**

| **Storage condition** | **Time point** | **Description** | **pH** | **Total impurity (%)** |
|---|---|---|---|---|
| Initial | | Clear colorless solution, free from visible particles | 7.3 | 0.36 |
| 40°C/75% RH | 1 month | Clear colorless solution, free from visible particles | 7.2 | 0.57 |
| | 3 month | Clear light yellow colored solution, free from visible particles | 7.2 | 0.86 |
| | 1 month | Clear colorless solution, free from visible particles | 7.2 | 0.44 |
| 25°C/60% RH | 3 month | Clear colorless solution, free from visible particles | 7.2 | 0.46 |

**Table 15. Stability profile of the pharmaceutical composition as mentioned in Example 5**

| **Storage condition** | **Time point** | **Description** | **pH** | **Total impurity (%)** |
|---|---|---|---|---|
| Initial | | Clear colorless solution, free from visible particles | 6.9 | 0.33 |
| 40°C/75% RH | 1 month | Clear light green colored solution, free from visible particles | 7.0 | 0.44 |
| | 3 month | Clear light green colored solution, free from visible particles | 6.9 | 0.70 |
| 25°C/60% RH | 1 month | Clear colorless solution, free from visible particle | 7.0 | 0.39 |
| | 3 month | Clear colorless solution, free from visible particle | 6.9 | 0.41 |

**Table 16. Stability profile of the pharmaceutical composition as mentioned in Example 6**

| **Storage condition** | **Time point** | **Description** | **pH** | **Total impurity (%)** |
|---|---|---|---|---|
| Initial | | Clear colorless solution, free from visible particles | 6.8 | 0.37 |
| 40°C/75% RH | 1 month | Clear colorless solution, free from visible particles | 6.8 | 0.72 |
| | 3 month | Clear light green colored solution, free from visible particles | 6.9 | 0.84 |
| | 6 months | Clear green colored solution, free from visible particles | 6.8 | 1.54 |
| | 1 month | Clear colorless solution, free from visible particle | 6.9 | 0.48 |
| 25°C/60% RH | 3 month | Clear colorless solution, free from visible particle | 6.9 | 0.44 |
| | 6 months | Clear light green colored solution, free from visible particles | 6.9 | 0.60 |

**Table 17. Stability profile of the pharmaceutical composition as mentioned in Example 7**

| **Storage condition** | **Time point** | **Description** | **pH** | **Total impurity (%)** |
|---|---|---|---|---|
| Initial | | Clear colorless solution, free from visible particles | 7.1 | 0.37 |
| 40°C/75% RH | 1 month | Clear colorless solution, free from visible particles | 7.4 | 0.68 |
| | 3 month | Clear light green colored solution, free from visible particles | 7.1 | 1.23 |
| 25°C/60% RH | 1 month | Clear colorless solution, free from visible particles | 7.2 | 0.46 |
| | 3 month | Clear colorless solution, free from visible particle | 7.1 | 0.62 |

**Table 18. Stability profile of the pharmaceutical composition as mentioned in Example 8**

| **Storage condition** | **Time point** | **Description** | **pH** | **Total impurity (%)** |
|---|---|---|---|---|
| Initial | | Clear colorless solution, free from visible particles | 7.4 | 0.34 |
| 40°C/75% RH | 1 month | Clear colorless solution, free from visible particles | 7.4 | 0.51 |
| | 3 month | Clear light green colored solution, free from visible particles | 7.3 | 0.82 |
| | 1 month | Clear colorless solution, free from visible particles | 7.4 | 0.38 |
| 25°C/60% RH | 3 month | Clear colorless solution, free from visible particle | 7.3 | 0.45 |

**Table 19. Stability profile of the pharmaceutical composition as mentioned in Example 9**

| **Storage condition** | **Time point** | **Description** | **pH** | **Total impurity (%)** |
|---|---|---|---|---|
| Initial | | Clear colorless solution, free from visible particles | 7.4 | 0.34 |
| 40°C/75% RH | 1 month | Clear colorless solution, free from visible particles | 7.4 | 0.61 |
| | 3 month | Clear light green colored solution, free from visible particles | 7.4 | 1.18 |
| 25°C/60% RH | 1 month | Clear colorless solution, free from visible particles | 7.4 | 0.40 |
| | 3 month | Clear colorless solution, free from visible particle | 7.3 | 0.50 |

**Table 20. Stability profile of the pharmaceutical composition as mentioned in Example 10**

| **Storage condition** | **Time point** | **Description** | **pH** | **Total impurity (%)** |
|---|---|---|---|---|
| Initial | | Clear colorless solution, free from visible particles | 6.9 | 0.35 |
| 40°C/75% RH | 1 month | Clear colorless solution, free from visible particles | 7.0 | 0.51 |
| | 3 month | Clear light green colored solution, free from visible particles | 7.0 | 0.90 |
| | 6 month | Light green colored solution, free from visible particles | 6.9 | 1.31 |
| 25°C/60% RH | 1 month | Clear colorless solution, free from visible particles | 6.9 | 0.34 |
| | 3 month | Clear colorless solution, free from visible particle | 6.9 | 0.48 |
| | 6 month | Clear colorless solution, free from visible particle | 7.2 | 0.60 |

**Table 21. Stability profile of the pharmaceutical composition as mentioned in Example 11**

| **Storage condition** | **Time point** | **Description** | **Total impurity (%)** |
|---|---|---|---|
| Initial | | Clear colorless solution, free from visible particles | 0.66 |
| 25°C/60% RH | 1 month | Clear colorless solution, free from visible particles | 0.64 |
| | 3 month | Clear colorless solution, free from visible particle | 0.75 |

**Table 22. Stability profile of the pharmaceutical composition as mentioned in Example 12**

| **Storage condition** | **Time point** | **Description** | **pH** | **Total impurity (%)** |
|---|---|---|---|---|
| Initial | | Clear colorless solution, free from visible particles | 7.5 | 0.55 |
| 40°C/75% RH | 1 month | Clear light green colored solution, free from visible particles | 7.3 | 1.86 |
| | 3 month | Clear light green colored solution, free from visible particles | 7.4 | 6.2 |
| 25°C/60% RH | 1 month | Clear light green colored solution, free from visible particles | 7.3 | 0.85 |
| | 3 month | Clear light green colored solution, free from visible particles | 7.5 | 1.94 |
| | 6 month | Clear light green colored solution, free from visible particles | 7.5 | 4.96 |

**Table 23. Stability profile of the pharmaceutical composition as mentioned in Example 13**

| **Storage condition** | **Time point** | **Description** | **pH** | **Total impurity (%)** |
|---|---|---|---|---|
| Initial | | Clear colorless solution, free from visible particles | 6.9 | 0.28 |
| 40°C/75% RH | 1 month | Clear light yellow colored solution, free from visible particles | 6.9 | 0.28 |
| | 3 month | Clear light green colored solution, free from visible particles | 7.1 | 0.98 |
| | 6 month | Clear green colored solution, free from visible particles | 6.9 | 2.20 |
| 25°C/60% RH | 1 month | Clear light yellow colored solution, free from visible particles | 7.0 | 0.21 |
| | 3 month | Clear light yellow colored solution, free from visible particles | 7.1 | 0.94 |
| | 6 month | Clear light green colored solution, free from visible particles | 6.9 | 1.57 |
| | 12 month | Clear light green colored solution, free from visible particles | 6.9 | 4.98 |

It is apparent from the above characterization data that injectable compositions comprising pemetrexed diacid, cyclodextrin, tromethamine are showing better room temperature stability results in comparison to formulations without these essential features.

Further to evaluate the room temperature stability of prior art formulation of pemetrexed disodium with cyclodextrins, the inventors have reproduced the prior art formulations, but found them to be less stable at room temperature in comparison to the compositions of present invention as shown below:

### Examples 14 and 15:

**Table 24. Prior art compositions of pemetrexed injection with HPβCD**

| **Ingredient** | **Example 14** | | **Example 15** | |
|---|---|---|---|---|
| | **Quantity per mL** | **Quantity %w/w** | **Quantity per mL** | **Quantity %w/w** |
| Pemetrexed disodium | 25 mg | 1 | 25 mg | 1 |
| HPβCD | 25 mg | 1 | 75 mg | 3 |
| Sodium hydroxide | q.s. | q.s. | q.s. | q.s. |
| Hydrochloric acid | q.s. | q.s. | q.s. | q.s. |
| Water for injection | q.s. to 1 mL | q.s. | q.s. to 1 mL | q.s. |

### - Manufacturing process (For Examples 14 and 15)

Suitable quantity of water in a manufacturing vessel was taken. Required quantity of HPβCD was added and dissolved. Once a clear solution was obtained, pemetrexed disodium was added and allowed to stir until clear solution was obtained. If required, the pH of solution was adjusted to 7.0±0.2 with the help of 1%w/v sodium hydroxide solution or 1%v/v hydrochloric acid solution. Volume was made up to 100% with water. The drug solution was filtered through a suitable 0.2µ PVDF filter. The filtered solution was filled into vials. The vials were stoppered and finally sealed.

**Table 25. Stability profile of the pharmaceutical composition as mentioned in Example 14**

| **Storage condition** | **Time point** | **Description** | **pH** | **Total impurity (%)** |
|---|---|---|---|---|
| Initial | | Clear colorless solution, free from visible particles | 7.2 | 0.58 |
| 40°C/75% RH | 1 month | Clear light green colored solution, free from visible particles | 6.8 | 5.21 |
| 25°C/60% RH | 1 month | Clear light green colored solution, free from visible particles | 6.9 | 1.50 |

**Table 26. Stability profile of the pharmaceutical composition as mentioned in Example 15**

| **Storage condition** | **Time point** | **Description** | **pH** | **Total impurity (%)** |
|---|---|---|---|---|
| Initial | | Clear colorless solution, free from visible particles | 7.1 | 0.34 |
| 40°C/75% RH | 1 month | Clear light green colored solution, free from visible particles | 7.1 | 1.79 |
| 25°C/60% RH | 1 month | Clear light green colored solution, free from visible particles | 6.9 | 0.68 |

## Claims

1. A room temperature stable liquid pharmaceutical composition for parenteral administration comprising:
a) pemetrexed diacid, wherein the concentration of pemetrexed diacid is from about 2.5 mg/ml to about 50 mg/ml;
b) tromethamine, wherein the concentration of tromethamine is from about 1 to 150 mg/ml; and
c) cyclodextrin, wherein the cyclodextrin is selected from β-cyclodextrin, hydroxypropyl-β-cyclodextrin, sulfobutylether-β-cyclodextrin, α-cyclodextrin and γ-cyclodextrin and the concentration of cyclodextrin is from about 40 to 500 mg/ml.

2. The composition according to claim 1, wherein the composition further comprises an inert gas, preferably wherein the inert gas is selected from nitrogen, argon and helium, preferably nitrogen.

3. The composition according to claim 1, wherein the molar ratio of pemetrexed diacid to cyclodextrin is in the range of 1:0.1 to 1:10, preferably 1:0.5 to 1:5, such as 1:2 to 1:5.

4. The composition according to any of the preceding claims, wherein the cyclodextrin is hydroxypropyl-β-cyclodextrin.

5. The composition according to any of the preceding claims, further comprising one or more pharmaceutically acceptable excipients selected from buffer, organic solvent, chelating agent, antioxidant and solubilizer, preferably wherein
(i) the buffer is selected from the group consisting of citrate, phosphate, arginate, acetate, glutamate, lactobionate, and a mixture thereof;
(ii) the organic solvent is selected from the group consisting of glycerol, poly ethylene glycol (PEG 300, PEG 400), propylene glycol (PG), ethanol, dimethyl acetamide (DMA) and a mixture thereof;
(iii) the chelating agent is selected from the group consisting of ethylenediaminetetraacetic acid (EDTA), sodium citrate and a mixture thereof;
(iv) the antioxidant is selected from the group consisting of methionine, sodium metasulphite, sodium bisulphite and a mixture thereof; and/or
(v) the solubilizer is selected from the group consisting of povidone (PVP), lecithine, sodium benzoate, poloxamer and a mixture thereof.

6. The composition according to any of the preceding claims for use in treating a subject suffering from malignant pleural mesothelioma and refractory non small cell lung cancer.

7. The composition according to any of the preceding claims, wherein the composition is ready to use or ready to be diluted with a suitable infusion solution before administration to the patient, preferably wherein the infusion solution is selected from saline or dextrose solution.

8. The composition according to claim 1, is substantially free from any particulate matter when present in a sealed container.

9. The composition according to any of the preceding claims comprising;
a) pemetrexed diacid preferably in an amount of 2.5 -50mg/ml,
b) tromethamine preferably in an amount of 15 to 35 mg/ml, and
c) hydroxypropyl-β-cyclodextrin preferably in an amount of about 200 mg/ml to about 300 mg/ml.

10. A process for manufacturing the liquid pharmaceutical composition for parenteral administration according to any of the preceding claims comprising the steps of:
a) purging inert gas in water for injection until the dissolved oxygen content of water is less than 7 mg/L, preferably less than 3 mg/L at room temperature,
b) dissolving cyclodextrin in water for injection of step a)
c) adding tromethamine to the above solution of step b),
d) adding and dissolving pemetrexed diacid to the above mixture at room temperature and optionally adjusting the pH of the solution to about 6.0 - 8.0.

11. A process according to claim 10, wherein the inert gas is nitrogen.

12. A process according to claim 10, wherein the cyclodextrin is hydroxypropyl-β-cyclodextrin.

13. A process for manufacturing the liquid pharmaceutical composition for parenteral administration according to claim 10 further comprising the additional following steps subsequent to steps a)-d):
e) filtering the solution and filling in vials,
f) blanketing the headspace of filled vials with nitrogen and
g) stoppering and sealing the vials.

## Patentansprüche

1. Bei Raumtemperatur stabile flüssige pharmazeutische Zusammensetzung zur parenteralen Verabreichung, die Folgendes umfasst:
a) Pemetrexeddisäure, wobei die Konzentration von Pemetrexeddisäure etwa 2,5 mg/ml bis etwa 50 mg/ml beträgt;
b) Tromethamin, wobei die Konzentration von Tromethamin etwa 1 bis 150 mg/ml beträgt; und
c) Cyclodextrin, wobei das Cyclodextrin ausgewählt ist aus β-Cyclodextrin, Hydroxypropyl-β-cyclodextrin, Sulfobutylether-β-cyclodextrin, α-Cyclodextrin und γ-Cyclodextrin und die Konzentration des Cyclodextrins etwa 40 bis 500 mg/ml beträgt.

2. Zusammensetzung nach Anspruch 1, wobei die Zusammensetzung ferner ein Inertgas umfasst, wobei das Inertgas vorzugsweise aus Stickstoff, Argon und Helium, vorzugsweise Stickstoff, ausgewählt ist.

3. Zusammensetzung nach Anspruch 1, wobei das molare Verhältnis von Pemetrexeddisäure zu Cyclodextrin im Bereich von 1:0,1 bis 1:10, vorzugsweise 1:0,5 bis 1:5, wie 1:2 bis 1:5, liegt.

4. Zusammensetzung nach einem der vorhergehenden Ansprüche, wobei das Cyclodextrin Hydroxypropyl-β-cyclodextrin ist.

5. Zusammensetzung nach einem der vorhergehenden Ansprüche, die außerdem einen oder mehrere pharmazeutisch annehmbare Hilfsstoffe enthält, die aus Puffer, organischem Lösungsmittel, Chelatbildner, Antioxidationsmittel und Lösungsvermittler ausgewählt sind, wobei vorzugsweise
(i) der Puffer ausgewählt ist aus der Gruppe bestehend aus Citrat, Phosphat, Arginat, Acetat, Glutamat, Lactobionat und einer Mischung davon;
(ii) das organische Lösungsmittel ausgewählt ist aus der Gruppe bestehend aus Glycerin, Polyethylenglykol (PEG 300, PEG 400), Propylenglykol (PG), Ethanol, Dimethylacetamid (DMA) und einer Mischung davon;
(iii) der Chelatbildner ausgewählt ist aus der Gruppe bestehend aus Ethylendiamintetraessigsäure (EDTA), Natriumcitrat und einer Mischung davon;
(iv) das Antioxidationsmittel ausgewählt ist aus der Gruppe bestehend aus Methionin, Natriummetasulfit, Natriumbisulfit und einer Mischung davon; und/oder
(v) der Lösungsvermittler ausgewählt ist aus der Gruppe bestehend aus Povidon (PVP), Lecithin, Natriumbenzoat, Poloxamer und einer Mischung davon.

6. Zusammensetzung nach einem der vorhergehenden Ansprüche zur Verwendung bei der Behandlung eines Patienten, der an malignem Pleuramesotheliom und refraktärem nicht-kleinzelligem Lungenkrebs leidet.

7. Zusammensetzung nach einem der vorhergehenden Ansprüche, wobei die Zusammensetzung gebrauchsfertig ist oder mit einer geeigneten Infusionslösung vor der Verabreichung an den Patienten verdünnt werden kann, wobei die Infusionslösung vorzugsweise aus Kochsalzlösung oder Dextroselösung ausgewählt ist.

8. Die Zusammensetzung nach Anspruch 1 ist im Wesentlichen frei von jeglichen Partikeln, wenn sie in einem verschlossenen Behälter vorliegt.

9. Zusammensetzung nach einem der vorhergehenden Ansprüche, die Folgendes umfasst:
a) Pemetrexeddisäure vorzugsweise in einer Menge von 2,5-50 mg/ml,
b) Tromethamin, vorzugsweise in einer Menge von 15 bis 35 mg/ml, und
c) Hydroxypropyl-β-cyclodextrin vorzugsweise in einer Menge von etwa 200 mg/ml bis etwa 300 mg/ml.

10. Verfahren zur Herstellung der flüssigen pharmazeutischen Zusammensetzung zur parenteralen Verabreichung nach einem der vorangehenden Ansprüche, das die folgenden Schritte umfasst:
a) Spülen von Inertgas in Wasser für Injektionszwecke, bis der Gehalt an gelöstem Sauerstoff im Wasser weniger als 7 mg/l, vorzugsweise weniger als 3 mg/l bei Raumtemperatur beträgt,
b) Auflösen von Cyclodextrin in Wasser für die Injektion von Schritt a)
c) Zugabe von Tromethamin zu der obigen Lösung aus Schritt b),
d) Hinzufügen und Lösen von Pemetrexeddisäure zu der obigen Mischung bei Raumtemperatur und gegebenenfalls Einstellen des pH-Werts der Lösung auf etwa 6,0 bis 8,0.

11. Verfahren nach Anspruch 10, wobei das Inertgas Stickstoff ist.

12. Verfahren nach Anspruch 10, wobei das Cyclodextrin Hydroxypropyl-β-cyclodextrin ist.

13. Verfahren zur Herstellung der flüssigen pharmazeutischen Zusammensetzung zur parenteralen Verabreichung nach Anspruch 10, das die folgenden zusätzlichen Schritte im Anschluss an die Schritte a)-d) umfasst:
e) Filtrieren der Lösung und Abfüllen in Fläschchen,
f) Überlagerung des Kopfraums der gefüllten Fläschchen mit Stickstoff und
g) Verschließen und Versiegeln der Fläschchen.

## Revendications

1. Composition pharmaceutique liquide stable à température ambiante pour une administration parentérale, comprenant :
a) du diacide de pémétrexed, dans laquelle la concentration en diacide de pémétrexed est d'environ 2,5 mg/ml à environ 50 mg/ml ;
b) de la trométhamine, dans laquelle la concentration en trométhamine est d'environ 1 à 150 mg/ml ; et
c) de la cyclodextrine, dans laquelle la cyclodextrine est choisie parmi la β-cyclodextrine, l'hydroxypropyl-β-cyclodextrine, la sulfobutyléther-β-cyclodextrine, l'α-cyclodextrine et la γ-cyclodextrine et la concentration en cyclodextrine est d'environ 40 à 500 mg/ml.

2. Composition selon la revendication 1, dans laquelle la composition comprend en outre un gaz inerte, de préférence dans laquelle le gaz inerte est choisi parmi l'azote, l'argon et l'hélium, de préférence l'azote.

3. Composition selon la revendication 1, dans laquelle le rapport molaire du diacide de pémétrexed à la cyclodextrine est compris dans la plage de 1:0,1 à 1:10, de préférence de 1:0,5 à 1:5, tel que de 1:2 à 1:5.

4. Composition selon l'une quelconque des revendications précédentes, dans laquelle la cyclodextrine est l'hydroxypropyl-β-cyclodextrine.

5. Composition selon l'une quelconque des revendications précédentes, comprenant en outre un ou plusieurs excipients pharmaceutiquement acceptables choisis parmi un tampon, un solvant organique, un agent chélateur, un antioxydant et un solubilisant, de préférence dans laquelle
(i) le tampon est choisi dans le groupe constitué par le citrate, le phosphate, l'arginate, l'acétate, le glutamate, le lactobionate, et un mélange de ceux-ci ;
(ii) le solvant organique est choisi dans le groupe constitué par le glycérol, le polyéthylène glycol (PEG 300, PEG 400), le propylène glycol (PG), l'éthanol, l'acétamide diméthylique (DMA) et un mélange de ceux-ci ;
(iii) l'agent chélateur est choisi dans le groupe constitué par l'acide éthylènediaminetétraacétique (EDTA), le citrate de sodium et un mélange de ceux-ci ;
(iv) l'antioxydant est choisi dans le groupe constitué par la méthionine, le métasulfite de sodium, le bisulfite de sodium et un mélange de ceux-ci ; et/ou
(v) le solubilisant est choisi dans le groupe constitué par la povidone (PVP), la lécithine, le benzoate de sodium, un poloxamère et un mélange de ceux-ci.

6. Composition selon l'une quelconque des revendications précédentes destinée à être utilisée dans le traitement d'un sujet souffrant d'un mésothéliome pleural malin et d'un cancer du poumon non à petites cellules réfractaire.

7. Composition selon l'une quelconque des revendications précédentes, dans laquelle la composition est prête à l'emploi ou prête à être diluée dans une solution de perfusion appropriée avant l'administration au patient, de préférence, la solution de perfusion est choisie parmi une solution saline ou une solution de dextrose.

8. Composition selon la revendication 1, qui est pratiquement exempte de toute particule lorsqu'elle se trouve dans un récipient scellé.

9. Composition selon l'une quelconque des revendications précédentes comprenant :
a) du diacide de pémétrexed de préférence en une quantité de 2,5 à 50 mg/ml,
b) de la trométhamine de préférence en une quantité de 15 à 35 mg/ml, et
c) de l'hydroxypropyl-β-cyclodextrine de préférence en une quantité d'environ 200 mg/ml à environ 300 mg/ml.

10. Procédé de fabrication de la composition pharmaceutique liquide pour une administration parentérale selon l'une quelconque des revendications précédentes comprenant les étapes consistant à :
a) purger le gaz inerte dans de l'eau pour injection jusqu'à ce que la teneur en oxygène dissous de l'eau soit inférieure à 7 mg/l, de préférence inférieure à 3 mg/l à température ambiante,
b) dissoudre la cyclodextrine dans l'eau pour l'injection de l'étape a)
c) ajouter de la trométhamine à la solution ci-dessus de l'étape b),
d) ajouter et dissoudre le diacide de pémétrexed dans le mélange ci-dessus à température ambiante et éventuellement ajuster le pH de la solution à environ 6,0 à 8,0.

11. Procédé selon la revendication 10, dans lequel le gaz inerte est l'azote.

12. Procédé selon la revendication 10, dans laquelle la cyclodextrine est l'hydroxypropyl-β-cyclodextrine.

13. Procédé de fabrication de la composition pharmaceutique liquide pour une administration parentérale selon la revendication 10 comprenant en outre les étapes suivantes supplémentaires après les étapes a) à d) :
e) filtrer la solution et remplir des flacons,
f) recouvrir l'espace de tête des flacons remplis avec de l'azote et
g) boucher et sceller les flacons.
